# EUROPEAN PATENT SPECIFICATION

(11) **EP 1 326 669 B1**
(45) Date of publication and mention of the grant of the patent: **09.08.2006**
(21) Application number: 01988603.5
(22) Date of filing: 26.09.2001
(51) Int. Cl.: A61M 15/00

(54) **INHALER**
INHALATOR
DISPOSITIF D'INHALATION

(30) Priority: 20.10.2000 GB 0025749
(43) Date of publication of application: 16.07.2003
(73) Proprietor: GLAXO GROUP LIMITED, Greenford, Middlesex UB6 ONN (GB)
(72) Inventor: BONNEY, Stanley George, Ware Herts SG12 ODP (GB); DAVIES, Michael Birsha, Ware, Herts SG12 0DP (GB)
(74) Representative: Rice, Jason Neale
(86) International application number: PCT/EP2001/011097
(87) International publication number: WO 2002/034319

(56) References cited:
- EP-A- 0 282 958
- WO-A-96/10459
- FR-A- 2 598 918
- US-A- 5 655 523
- US-A- 5 694 920
- US-A- 5 941 240

## Description

### Technical Description

This invention relates to an inhaler for dispensing dry powder medicaments. In particular, the invention relates to an inhaler that does not require manual actuation by a patient.

### Background to the Invention

Medical dispensers are well known for the dispensing of various kinds of medicament. Inhalation devices, such as metered dose inhalers (MDI) and dry powder inhalers are known for the delivery of medicament for the treatment of respiratory disorders such as asthma and chronic inflammatory pulmonary disease.

There are a number of different dry powder inhalers presently available. In one instance, the drug is encapsulated in hard gelatine and the inhaler comprises a device for perforating a capsule prior to the patient inhaling the contents. After the patient manually activates the opening of the capsule, a cloud of dry particles is directed into the nose or mouth of the patient usually by a channelling device such as a cylinder or open-ended cone. Concurrently with the release of the capsule contents, the patient inhales the drug particles into the lungs or nasal cavity. The vacuum created by the patient on inhalation is intended to empty the capsule contents.

The inhaler exemplified in EP-A-467172 accommodates a blister pack wherein each blister retains a dose of medicament in dry powder form. When a blister is positioned for dosing, a mechanism within the inhaler punctures the blister, releasing the contents for inhalation by the user as described *supra.* US-A-4805811 discloses a dry powder inhaler comprising a dry powder reservoir from which a dosing plate having a number of dosing "cups" is filled from the reservoir prior to inhalation. As with the examples described *supra,* this device requires manual metering and/or releasing of a metered dose prior to inhalation.

It may be understood that effective delivery of medicament to the patient using an inhalation device as described is to an extent dependent on the patient's ability to manually actuate the device (e.g. puncturing of a capsule) and to co-ordinate the actuation thereof with the taking of a sufficiently strong inward breath. For some patients, particularly young children, the elderly and the arthritic, manual actuation of the device can present difficulties. Other patients find it difficult to co-ordinate the taking of a reliable inward breath with actuation of the device. Both of these sets of patients run the risk that they do not receive the appropriate dose of medicament.

US-A-5239992 discloses a loose powder inhaler wherein the vacuum created on inhalation by the user drives a dosing piston to measure and liberate a dose concurrent with inhalation of the drug. However, this device is reliant on the patient being able to draw sufficient breath to create the necessary vacuum and therefore does not alleviate the problems discussed *supra.*

As further background prior art there may be mentioned EP-A-0282958, US-A-5941240, FR-A-2598918, US-A-5655523, US-A-5694920 and WO-A-96/10459.

The Applicants have now developed a dry powder inhaler that does not require manual actuation by the patient.

### Summary of the Invention

Accordingly, the invention provides an inhaler according to claim 1.

Metering of the medicament is wholly dependent on the actuation of the breath sensor by the patient's breath. Accordingly, the medicament is protected from unintentional manual actuation of the dispenser whereby the dose may be lost or exposed to the environment.

Metering of the dry powder medicament immediately prior to inhalation has a number of advantages. Firstly, the medicament has no time to absorb moisture from its environment outside the dry powder reservoir. Also, the problem of medicament adhesion or sticking to the metering mechanism is alleviated or substantially reduced.

The amount of dry powder may be measured on a volume or weight basis.

Typically, the meter comprises a volume and/or a weight and/or time and/or surface area and/or a particle counting regulated mechanism.

In one embodiment, metering of medicament dose may be achievable by pulsing electrical current flow through the meter for a selected dispensing time.

For example, the meter may comprise a valve (for example, a linear or rotary valve) and/or a piston and/or a load cell. In another aspect, the dose-metering mechanism may comprise a plunger, such as might exist in a syringe. Embodiments including multiple plungers and multiple syringe chambers are also envisaged.

Preferably, the meter comprises at least one metering chamber. In one embodiment, on actuation of the meter, the or each metering chamber moves into fluid communication with the reservoir.

in one embodiment, the meter and the reservoir are relatively rotatable with respect to each other about a common central axis. Preferably, the or each metering chamber is adapted to be in fluid communication selectively with the reservoir or with the patient.

The or each metering chamber may have a variable volume. Alternatively, the or each metering chamber may have a fixed volume which is variable by insertion of a plunger or piston. The or each metering chamber may be formed from expandable material and/or have a telescopic or concertina arrangement.

In one embodiment, the inhaler further comprises a gas permeable dry powder retaining means below the or each metering chamber. The retaining means may be made from a gas-permeable filter, a mesh screen, a porous material or a perforated chamber element.

A reset mechanism may be provided for resetting the meter after actuation thereof. The reset means may for example, comprise a spring, motor, or other mechanical arrangement, and/or an electronic arrangement.

In a preferred aspect, the inhaler further comprises transport means to transport the metered volume from the reservoir to a delivery position. Preferably, the transport means is actuable by the meter.

As used herein, the term "dose-release means" refers to the means for the making available of the dose for release to the patient, and the actual dispensing to the patient.

Preferably, the release means is actuable by the coupling means and/or the meter and/or the transport means.

Typically, the breath sensor and/or the meter and/or the transport means actuates the release means immediately after, or concurrent with, the actuation of the meter.

In this embodiment, the invention ensures that only after a dose has been metered from the dry powder reservoir can the medicament be made available for inhalation by the patient. Accordingly, the metered dose does not remain waiting in a metering chamber or delivery unit or release chamber for any length of time and therefore there is substantially reduced or alleviated the chance of deposition or sticking of the medicament onto the walls of the device, or the chance of moisture ingress or contamination from the external environment.

The release is active in the sense that medicament is actively dispensed from the container.

As the release means is active and comprises means to propel pressurised gas in the direction of patient inhalation, the patient receives a positive signal that the dose has been dispensed which may add to patient confidence. An active release means may also increase the efficacy of delivery of the medicament, for example, the drug may be released in a more focussed plume or cloud towards the back of the inhaler's nose or throat.

Preferably, the gas-propelling means provides at least one pulse of gas on actuation.

The gas-propelling means may provide one pulse of gas for each dose dispensed.

The gas may be air or an inert gas.

In another embodiment, the inhaler additionally comprises climate control means. Preferably, the climate control means is actuable by the coupling means and/or the meter and/or the transport means and/or the release means.

The climate control means may comprise means to (i) reduce moisture increase in the inhaler; and/or (ii) maintain ambient temperature; and/or (iii) dry the meter prior to actuation of the inhaler.

The climate control means may comprise a desiccant and/or a heater.

The heater may dry the meter prior to metering of the dose and/or immediately after the dose is dispensed.

The climate control means may comprise a temperature and/or a moisture sensor.

The coupling means may comprise a spring and/or a lever. Alternatively, or in addition, the coupling means may comprise a solenoid.

In one embodiment, the coupling means is reversibly deformable in response to heating thereof or application of a magnetic field thereto.

The inhaler may additionally comprise a reset coupling which is reversibly deformable in response to heating thereof or application of a magnetic field thereto.

Preferably, heating is achievable by electric current flow through the coupling or reset coupling.

Preferably, the coupling or reset coupling comprises a wire, strip, coil or tube.

Arrangements comprising multiple strips, wires, coils, or tubes are also envisaged. The multiple strips, wires, coils, or tubes may be arranged in any suitable fashion including parallel or series arrangements and bundle arrangements.

In one particular aspect, the coupling or reset coupling comprises one or more wires which contract in response to heating or application of a magnetic field thereto.

Preferably, the degree of contraction of the coupling is from 2% to 8%.

In one embodiment, the coupling comprises an alloy which undergoes a phase transition on heating (shape memory alloys). Certain shape memory alloys also undergo a change in shape on re-cooling. Such two way shape memory alloys are also envisaged for use herein.

In one embodiment, the shape memory alloy is preferably a nickel-titanium alloy such as a nickel-titanium alloy comprising from 5% to 95%, preferably from 20% to 80%, nickel by weight and from 95% to 5%, preferably from 80% to 20%, titanium by weight. By nickel-titanium alloy it is meant an alloy comprised essentially of nickel and titanium, although other elements such as Cu and Nb may be present in small (e.g. trace) amounts.

In other embodiments, the shape memory alloy is preferably a copper-aluminium-nickel alloy or a copper-zinc-aluminium alloy. Trace amounts of other elements may also be present.

In further embodiments, the coupling comprises an alloy which undergoes a phase transition on application of a magnetic field thereto (magnetic shape memory alloys). These materials are generally intermetallic, ferromagnetic alloys that exhibit twin variants in the martensitic, or low-temperature, phase of the material. Suitable magnetic shape memory alloys are for example, described in US Patent No. 5,958,154.

In one embodiment, the magnetic shape memory alloy exhibits an austenitic crystal structure above a characteristic phase transformation temperature and also exhibits a martensitic twinned crystal structure below the phase transformation temperature. The alloy has a magnetocrystalline anisotropy energy that is sufficient to enable motion of twin boundaries of the martensitic twinned crystal structure in response to application of a magnetic field to the martensitic twinned crystal structure.

Where a magnetic shape memory alloy is employed the inhaler preferably includes a magnetic field source disposed with respect to the coupling in an orientation that applies to the coupling a magnetic actuation field in a direction that is substantially parallel with a selected twin boundary direction of the martensitic twinned crystal structure of the coupling material.

Alternatively, the inhaler preferably includes a magnetic bias field source disposed with respect to the coupling in an orientation that applies a magnetic bias field to the coupling, and a magnetic actuation field source disposed with respect to the coupling in an orientation that applies a magnetic actuation field to the coupling material in a direction that is substantially perpendicular to the orientation of the applied magnetic bias field.

A preferred magnetic shape memory alloy is the actuator material comprising an alloy composition defined as Ni_{65-x-y}Mn₂₀+xGa₁₅+y, where x is between 3 atomic % and 15 atomic % and y is between 3 atomic % and 12 atomic %. Preferably, the actuator material comprises an alloy composition defined as Ni_{65-x-y}Mn₂₀+xGa₁₅+y, where x is between 6 atomic % and 10 atomic % and y is between 5 atomic % and 9 atomic %; or where x is between 12 atomic % and 15 atomic % and y is between 3 atomic % and 6 atomic %; or where x is between 10 atomic % and 14 atomic % and y is between 3 atomic % and 6 atomic %; or where x is between 7 atomic % and 11 atomic % and y is between 3 atomic % and 7 atomic %. In a particularly preferred aspect, the alloy is Ni₅₀Mn₂₅Ga₂₅.

Another preferred magnetic shape memory alloy is the alloy having the composition (NiₐFe_{b}Co_{c})_{65-x-y}(Mn_{d}FeₑCo_{f})₂₀+x(Ga_{g}SiₕAlᵢ)₁₅+y, where x is between 3 atomic % and 15 atomic % and y is between 3 atomic % and 12 atomic %, and where a+b+c=1, where d+e+f=1, and g+h+i=1.

In preferred aspects, b is between zero and 0.6, c is between zero and 0.6, and e, f, h and i are each zero; or b and c are each zero, e is between zero and 0.6, f is between zero and 0.6, and h and i are each zero; or b, c, e and f are each zero, h is between zero and 0.5, and i is between zero and 0.5.

Preferably, the one or more wires have a diameter from 30 to 400 micrometers, preferably from 50 to 150 micrometers.

Preferably, the coupling comprises from two to twelve, preferably six to ten wires which contract in response to heating or application of a magnetic field thereto.

The wires may be arranged in any suitable fashion including parallel or series arrangements and bundle arrangements.

In another aspect, the coupling comprises a strip which comprises multiple layers of different metals. Suitable strips typically comprise a plurality of layers of material, each material having a different coefficient of thermal expansion.

Preferred examples of strips include those comprising multiple layers of different metals (e.g. bimetallic strips) and strips comprising at least one piezoelectric material. Suitable piezoelectric materials include piezoelectric ceramics, such as compounds of lead zirconate and lead titanate, and piezoelectric crystals which are generally polycrystalline ferroelectric materials with the perovskite structure.

In one aspect, the coupling is deformable in response to heating arising from electrical current flow in the range from 0.01A to 100A, preferably from 0.1A to 5A. Alternatively, the coupling is deformable in response to heating arising from the application of an electrical voltage, particularly where the coupling comprises a piezoelectric material.

In another aspect, the coupling is deformable in response to a magnetic field of from 0.01 to 100 Tesla. The magnetic field may for example, be produced by a permanent magnet or by an electromagnet.

The deformation of the coupling (e.g. by electrical current flow therethrough) may be responsive to the detection of the inward breath of a patient. Alternatively, deformation of the coupling (e.g. by electrical current flow therethrough) may be responsive to a trigger coupled to any point in the breathing pattern of the patient, such as the end of the outward breath.

As used herein the term breath sensor encompasses any suitable means for monitoring, measuring, tracking or indicating the breath of a patient and may comprise one or more sensors.

Preferably, the breath sensor electro-mechanically actuates the meter at a predetermined trigger point in the patient's breath cycle. For example, the trigger point may be during the inhalation or exhalation stage of the patient's breath cycle.

In one aspect, the sensor comprises a breath-movable element which is movable in response to the breath of a patient. Preferably, the breath-movable element is selected from the group consisting of a vane, a sail, a piston, a diaphragm and an impeller.

Movement of the breath-movable element may be detectable by any suitable technique for detecting movement. Suitable techniques include optical detectors, magnetic detectors or detectors using detection of capacitative effects.

Optical detectors may be used to detect movement of the breath-movable element by providing the element with a patterned outer surface, for example strips in a barcode type arrangement, and locating the optical detector so that it points towards the patterned surface. Movement of the breath-movable element alters the amount of the light source which reflects back onto the optical detector as the beam passes over the patterned surface. The strips may be arranged so that the direction of movement of the element can be detected.

Magnetic detectors may be used to detect the movement of breath-movable element by the use of a magnetic switch device. A reader is located on the dispenser and magnetic material embedded within the breath-movable element (or vice-versa). Movement of the breath-movable element results in a change of the magnetic field experienced by the reader. Alternatively, a Hall effect device can be used whereby a semiconductor measures the strength of the magnetic field of the magnetic material on the breath-movable element.

Detection of capacitative effects may be used to detect movement of the breath-movable element by adding a conductive part to the element and also to a second fixed part of the dispenser. Movement of the breath-movable element results in a change in capacitance which can be measured.

In another aspect, the sensor comprises a pressure sensor for sensing the pressure profile associated with the breath of a patient. A pressure transducer is an example of a suitable pressure sensor.

In another aspect, the sensor comprises an airflow sensor for sensing the airflow profile associated with the breath of a patient.

In another aspect, the sensor comprises a temperature sensor for sensing the temperature profile associated with the breath of a patient.

In another aspect, the sensor comprises a moisture sensor for sensing the moisture profile associated with the breath of a patient.

In another aspect, the sensor comprises a gas sensor for sensing the chemical profile, for example, the oxygen or carbon dioxide profile associated with the breath of a patient.

Preferably, the sensor is connectable to an electronic information processor. The connection may be direct or via any suitable mechanical or electronic transfer means.

Preferably, the electronic information processor actuates the meter at a predetermined trigger point in the breath cycle.

Preferably, the electronic information processor includes an active memory for storing information about the breath cycle.

Suitably, the electronic information processor includes a predictive algorithm or look-up table for predicting the optimum trigger point. For example, a real-time analysis of the patient waveform may be made and the optimum trigger point derived by reference to that analysed waveform.

Suitably, the electronic information processor includes a second predictive algorithm or look-up table for predicting the optimum amount of medicament to release. Suitably, the electronic information processor includes a dose memory for storing information about earlier delivered doses and reference is made to the dose memory in predicting the optimum amount of medicament to release.

Preferably, the inhaler additionally comprises a display for displaying information about the optimum amount of medicament to release.

Preferably, the inhaler according additionally comprises a selector for selecting the amount of medicament to release.

In one aspect, the selector is manually operable.

Alternatively or in addition, the selector is operable in response to a signal from the electronic information processor.

Preferably, the selector comprises a timing mechanism for varying the time interval of actuation of the meter and/or dose-release mechanism.

The selector may comprise a multiple-fire mechanism for multiple actuation of the inhaler wherein each actuation releases a portion of the optimum amount of medicament.

Preferably, the inhaler additionally comprises an electrical energy source. In one aspect, the electrical energy source comprises a voltaic cell or battery of voltaic cells which may be rechargeable. In another aspect, the electrical energy source comprises a photovoltaic cell or battery of photovoltaic cells. The additional energy source may be mechanically-generated, for example, the energy source may comprise a biasable resilient member, e.g. a spring. Therefore, the electrical energy source may comprise a converter for converting mechanical energy into electrical energy.

The energy source may comprise a source of compressed fluid, preferably compressed gas, or a chemical energy store, preferably a chemical propellant or ignition mixture. Other sources may include physical explosives such as liquefied or solidified gas in a canister which burst when heated or exposed to the atmosphere.

Any electrical circuit may incorporate voltage amplification means for generating a higher voltage than that supplied by the voltaic cell or battery of voltaic cells, for example a step-up or inverting switching circuit or a dc-dc converter incorporating an oscillator, transformer and rectifier.

The electrical circuit may incorporate one or more energy storage components such as capacitors or inductors in order to supply a high enough instantaneous current to raise the temperature of the strips or wires at the required rate to the required temperature.

The input to the electrical circuit may be connected to the electrical energy source by means of a mechanical, electro-mechanical or electronic switching component.

The output of the electrical circuit may be connected to the strips or wires or to an electromagnet by means of a mechanical, electro-mechanical or electronic switching component or by a component allowing the output current to be controlled in a linear or digital (e.g. pulse width modulated) manner.

The strip or wire components may be powered from the battery using a switching component without additional power supply circuitry.

Suitably, the inhaler additionally comprises a controller for controlling the amount of electrical current flow through the coupling or to an electromagnet.

Suitably, the inhaler additionally comprises a timer for controlling the duration of electrical current flow through the coupling or to an electromagnet.

Suitably, the inhaler additionally comprises a local electrical store such as a capacitor or inductor.

Suitably, the inhaler is provided with a manual override to enable actuation of the device in the event of loss of electrical power. For example in the event of an emergency or system failure.

Preferably, the inhaler includes a safety mechanism to prevent unintended multiple actuations of the device. The patient is thereby protected from inadvertently receiving multiple doses of medicament in a situation where they take a number of short rapid breaths. More preferably, the safety mechanism imposes a time delay between successive actuations of the device. The time delay is typically in the order of from three to thirty seconds.

Preferably the inhaler comprises an actuation or dose counter for counting the number of actuations of the meter or dose-release means or releases of dose therefrom. More preferably, counting will occur even if the metering and/or release means is manually actuated, that is, the actuation counter is independent of the coupling between the breath sensor and the dose-dispensing means.

The actuation counter may be mechanical or electronic.

Suitably, the inhaler is provided with child-resistance features to prevent undesirable actuation thereof by a young child.

The inhaler of the invention is suitable for dispensing medicament, particularly for the treatment of respiratory disorders such as asthma and chronic obstructive pulmonary disease (COPD).

Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate, ketotifen or nedocromil; antiinfectives e.g., cephalosporins, penicillins, streptomycin, sulphonamides, tetracyclines and pentamidine; antihistamines, e.g., methapyrilene; anti- inflammatories, e.g., beclomethasone dipropionate, fluticasone propionate, flunisolide, budesonide, rofleponide, mometasone furoate or triamcinolone acetonide; antitussives, e.g., noscapine; bronchodilators, e.g., albuterol, salmeterol, ephedrine, adrenaline, fenoterol, formoterol, isoprenaline, metaproterenol, phenylephrine, phenylpropanolamine, pirbuterol, reproterol, rimiterol, terbutaline, isoetharine, tulobuterol, or (-)-4-amino-3,5-dichloro-α-[[[6-[2-(2-pyridinyl)ethoxy] hexyl]methyl] benzenemethanol; diuretics, e.g., amiloride; anticholinergics, e.g., ipratropium, tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Medicaments can also be delivered in combinations. Preferred formulations containing combinations of active ingredients contain salbutamol (e.g., as the free base or the sulphate salt) or salmeterol (e.g., as the xinafoate salt) in combination with an antiinflammatory steroid such as a beclomethasone ester (e.g., the dipropionate) or a fluticasone ester (e.g., the propionate). A particularly preferred combination comprises salmeterol xinafoate salt and filuticasone propionate.

Preferred medicaments are selected from albuterol, salmeterol, fluticasone propionate and beclomethasone dipropionate and salts or solvates thereof, e.g., the sulphate of albuterol and the xinafoate of salmeterol, and any mixtures thereof. Alternatively, the dispenser may be employed for dispensing vaccine.

Indeed, it is envisioned in accordance with this invention that any suitable diagnostic, prophylactic or therapeutic agent can used with the inhaler herein. Generally, drug particles suitable for delivery to the bronchial or alveolar region of the lung have an aerodynamic diameter of less than 10 micrometers. Other sized particles may be used if delivery to other portions of the respiratory tract is desired, such as the nasal cavity, mouth or throat. The medicament may be a pure drug, but more appropriately, it is preferred that powder comprise a drug mixed with a bulking agent (excipient), for example, lactose.

Additional powders may be engineered with particular densities, size ranges, or characteristics. Particles may comprise active agents, surfactants, wall forming materials. or other components considered desirable by those of ordinary skill.

Blends of bulking agents and drugs are typically formulated to allow the precise metering and dispersion on the powder into doses. A standard blend, for example, contains 13000 micrograms lactose mixed with 50 micrograms drug, yielding an excipient to drug ratio of 260:1. Because the present invention can meter and dispense such blends more accurately and effectively, dosage blends with excipient to drug ratios of 60:1, and potentially 2:1, may be used. At very low blend levels, however, the drug dose reproducibility becomes more variable Typically, the dry powder medicament includes a pharmaceutical excipient in dry powder form.

In one embodiment, the density of the dry powder medicament particles is reduced relative to standard dry powder medicament.

In another embodiment, the dry powder medicament particles are aerodynamically shaped to improve medicament delivery to the patient.

### Brief Description of the Drawings

The invention will now be described further with reference to the accompanying figures in which:
Figure 1 shows a typical patient inhalation profile of airflow (litres par minute) against time (seconds) as a patient inhales using a medicament dispenser according to the invention;
Figure 2 shows a flow diagram of the sequence of events during the dispensing of a dose of medicament to a patient, wherein the inhaler includes a heater to dry the meter apparatus according to one aspect of the invention;
Figure 3 shows a metering mechanism for a medicament dispenser according to one aspect of the invention wherein metering is by volume;
Figure 4 shows a metering mechanism for a medicament dispenser according to another aspect of the invention wherein metering is by weight;
Figure 5 shows a metering mechanism for a medicament dispenser according to another aspect of the invention wherein metering is by surface area;
Figure 6 shows a metering mechanism for a medicament dispenser according to another aspect of the invention wherein metering is by time; and
Figure 7 shows a medicament dispenser having a metering mechanism as shown in Figure 3 and an associated system diagram linking the transport means to an electromechanical coupling according to one aspect of the invention.

### Detailed Description of the Drawings

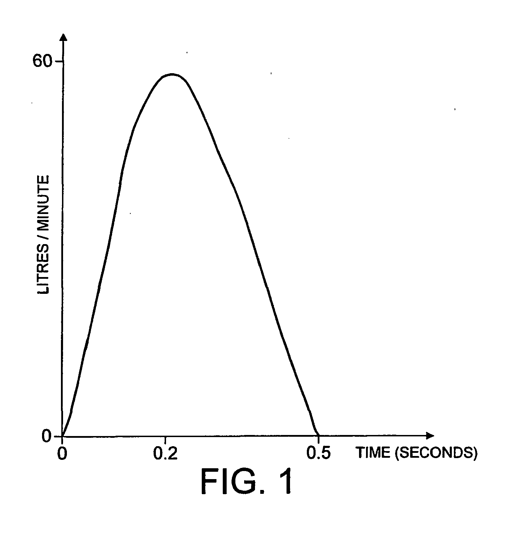

Referring now to the figures, a typical inhalation profile of an adult patient is illustrated in Figure 1. In this example, the maximum airflow is 60 litres min⁻¹, approximately 200ms after the breath is initiated. Optimally, a medicament dispenser responsive to a breath sensor should complete the dispensing cycle and make available the medicament for inhalation before the end of the patient's breath cycle.

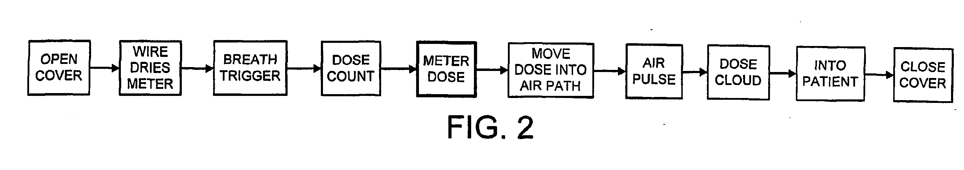

Figure 2 illustrates the sequence of events during the use of a medicament dispenser in the form of a dry powder inhaler. If the inhaler comprises a protective cover, the cover is removed to expose a mouthpiece. Opening the protective cover activates a heating element to dry a metering pocket in the inhaler and thus alleviate any problems associated with condensation or general environmental moisture. After the patient starts to inhale, a breath sensor is activated and a flow sensor actuates the metering of the medicament from a medicament reservoir at a threshold airflow value. A transport means then transports the dose from a non-dispensing position to a delivery position wherein the medicament is ready for inhalation by the patient. Aerosolisation means in the form of an air pulse generator produces a dose cloud directed towards the patient through the mouthpiece so that the patient may sense the dose entering the mouth and thus the target airways. Notably, the patient has not had to provide any manual intervention throughout the entire metering and dispensing sequence.

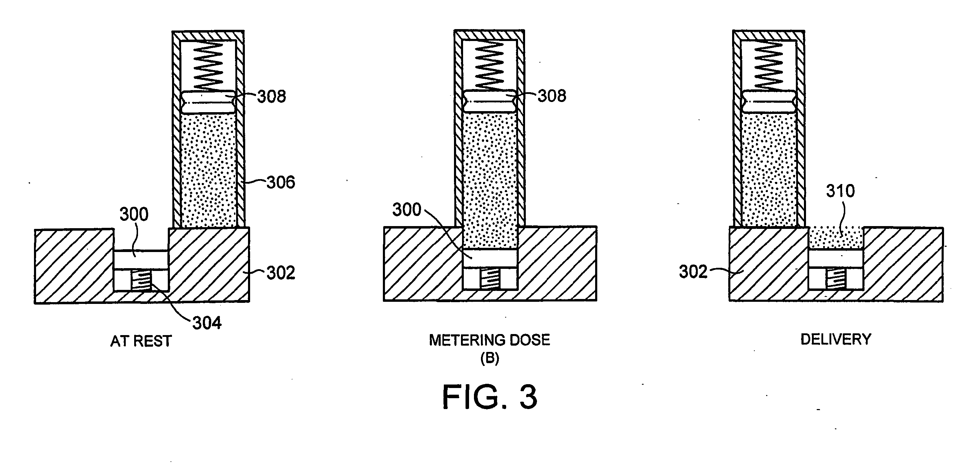

Figure 3 illustrates a volumetric metering mechanism for use inside a medicament dispenser according to one aspect of the invention. A metering plate 300 is positioned in a dose plate 302 and is sited on an adjustable screw 304 for altering the volume of dose to be metered as required. The adjustable screw 304 may be driven by an ultrasonic motor (not shown). A medicament reservoir 306 is movable relative to the dose plate 302. In the metering position (B) the reservoir 306 is positioned directly over the metering plate 300 and the medicament 310 is dispensed onto the metering plate 300. Metering may occur by gravitational force. However, in this case a spring loaded plunger 308 is sited in the medicament reservoir 306 to maintain a constant pressure on the medicament throughout the life of the device.

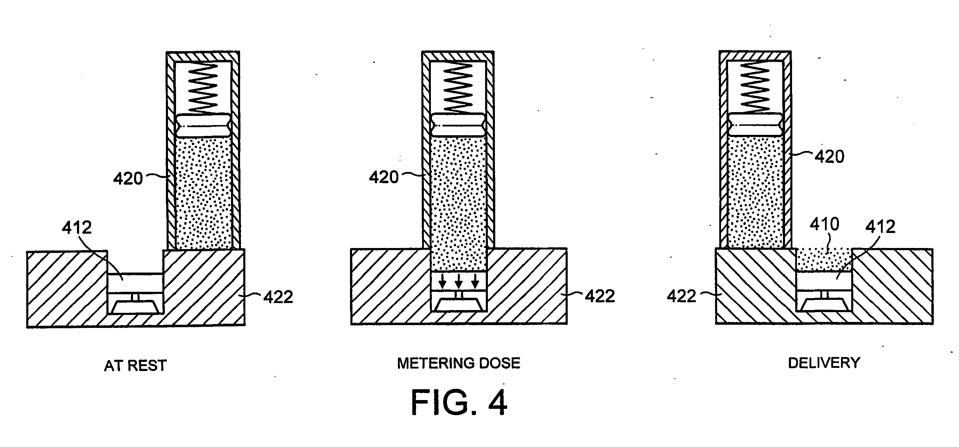

Metering may be by weight as shown in Figure 4. In this example, the metering plate of Figure 3 is replaced by a load cell 412 which measures the amount of medicament on its surface. Once the required weight of medicament 410 is dispensed from the reservoir 420, the reservoir 420 and/or the dose plate 422 are moved relative to each other to transport the metered dose to a delivery position.

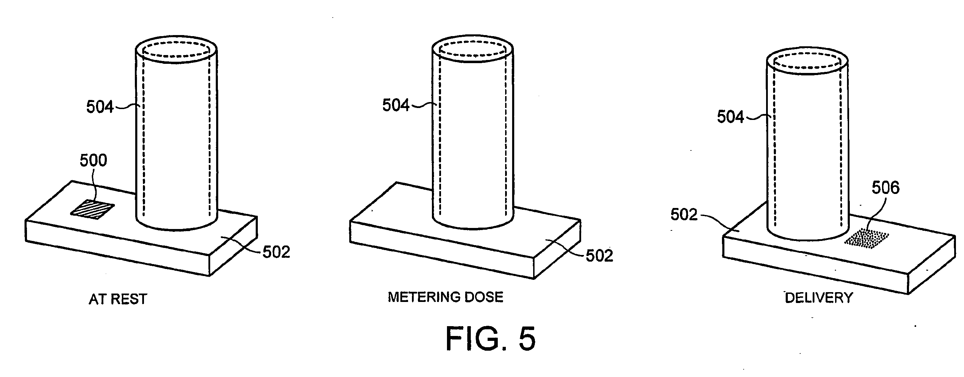

Metering may be by surface area as illustrated in Figure 5. In this case, a defined metering area 500 is sited on the dose plate 502. Prior to metering of the medicament, the metering plate 500 is electrostatically charged so that it attracts medicament particles. After actuation of the metering mechanism, the metering area 500 is moved into direct communication with the medicament reservoir 504. Medicament particles 506 within the medicament reservoir 504 are electrostatically charged with an opposite polarity to the metering area 500. Thus, a monolayer of particles 506 is attracted to the area 500. Movement of the reservoir 504 away from the metering area 500 reveals a metered dose of particles 506. In one embodiment, after metering, the polarity of the particles in the reservoir 504 or the area 500 may be reversed so as to stop further attraction.

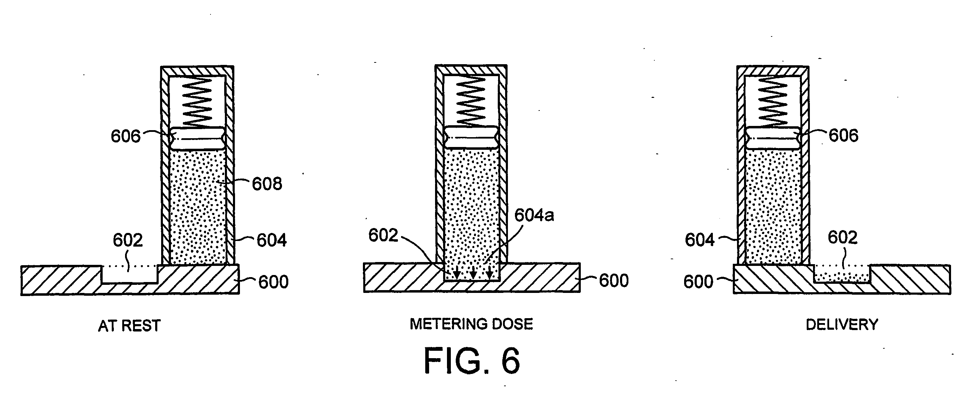

Figure 6 illustrates a metering mechanism using time. A dose plate 600 has a metering cavity 602. A medicament reservoir 604 contains a spring-tensioned plunger 606 to maintain a constant pressure on the contents 608 of the medicament reservoir 604. In order to meter the medicament, the dose plate 600 is moved relative to the open end 604a of the reservoir 604 at a defined speed so that the medicament flows into the cavity 602 at a constant flow rate. The amount of metered medicament will be measured as a function of the time the cavity 602 is exposed to the reservoir outlet 604a.

Metering may also be achieved by particle counting. In this case, the particles are optically counted by lasers as they flow into the cavity 602. The reservoir 604 is moved away from the cavity 602 after a predetermined number of medicament particles has flowed therein.

In another embodiment, threaded screw means may be used to meter the medicament.
Although the Figures 3 to 6 show only one metering chamber, it can be envisaged that there may be a plurality of metering chambers and further, actuation of the inhaler may result in the metering and transporting of medicament in more than one chamber to the patient. In this case the inhaler will further comprise a dose controlling means. Thus the invention is relevant to unit dose inhalers, single-dose inhalers, and multi-dose inhalers.

The inhaler may also comprise a heater (not shown), in the form of a wire, to dry a dose plate prior to metering a dose of medicament. This has the advantage of removing any moisture from the dose plate that might adversely affect the metering of a dose. The heater may be triggered either immediately prior to metering a dose, immediately post metering a dose, or immediately after a dose has been dispensed.

Movement of, *inter alia,* the dose plate and/or the medicament reservoir, may be driven by a DC motor, a piezo-electric motor (e.g. an ultrasonic motor), and/or shape memory alloy (SMA) wires. For example, a DC motor may transform electrical energy from a power supply actuated by the patient's breath, to rotary and/or linear motion through gearing means and/or rack and pinion means. A piezo-electric motor (e.g. an ultrasonic motor) may drive rotary and/or linear motion. SMA wires may drive linear motion as activation of an electrical current through the wires causes the wires to contract in length.

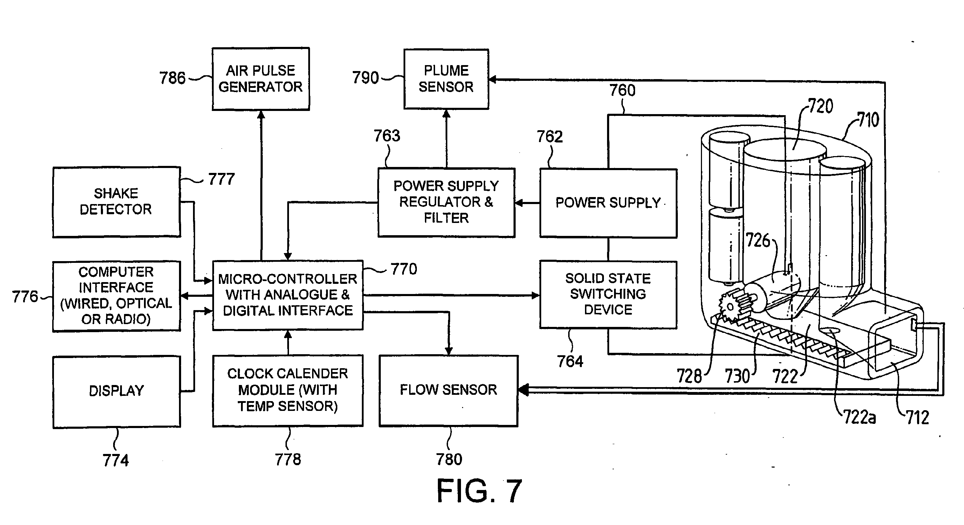

Figure 7 shows a schematic representation of a breath-operable medicament dispensing system. The system comprises a metered dose inhaler similar according to the invention comprising a tubular housing 710 having a dispensing outlet 712 in the form of a mouthpiece. Within the housing 710 sits a medicament reservoir which has a volumetric dose-metering mechanism as illustrated in Figure 3. A slide plate 722 is transportable between a metering position X and a delivery position Y enabling the passage of dispensed dose in a medicament pocket 722a to the dispensing outlet 712.

A DC motor 726 drives a rotary gear wheel 728 which in turn drives the slide plate 722 by the rack 730. Control of electrical current flow to the DC motor 726 is achievable using the illustrated circuitry. The DC motor 726 is connected to actuation circuit 760 which includes a power supply 762 (e.g. a voltaic cell or battery of voltaic cells) and a switch 764 in the form of a solid state switching device. The switch 764 itself connects to control circuitry including micro-controller 770 which has an analogue and/or digital interface. The power supply for the control circuitry is taken from the power supply 762 after suitable regulation and filtering 763. The micro-controller 770 itself connects with a flow sensor 780 which is associated with a breath sensor 790.

It may be appreciated that current flow to the DC motor 726 and hence actuation of the transport means 722 may be achievable as follows. The patient inhales through the mouthpiece 724 resulting in a change in airflow within the housing 710. The change in airflow is detected by the flow sensor 780 which sends a signal to the micro-controller 770. The micro-controller 770, in turn sends a switching signal to the solid state switching device 764 which results in closing of the actuation circuit and electrical current flow therethrough. DC motor 726 thus drives the slide plate 722 to a metering position and then to a delivery position and hence, dispensing of the medicament to the inhaling patient.

It may also be seen in Figure 7 that the micro-controller 770 is connected to a display 774 for display of Information to the patient and also with a computer Interface 776 for exchange of data therewith. Communication with the computer interface 776 may be via a wired, optical or radio communications link. The micro-controller 770 is may also be connected to shake detector 777 for use in detecting whether the container 720 is shaken prior to actuation of the transport means 722 and to a clock-calendar module 778 including a temperature sensor. All circuitry and components thereof Including the power supply 762, display 774, shake detector 777, computer interface 776 and clock-calendar module 778 may be arranged to be present on the housing 710 such that the system is in the form of a discrete, hand-held device.

In addition, the micro-controller 770 is linked to an air pulse generator 786 for actuating the release mechanism for aerosolisation of the dose. The power supply 763 is connected to a plume sensor 790 which senses when a dose of medicament leaves the dispenser and feeds back to turn off the power supply.

It may be appreciated that any of the parts of the inhaler or actuator which contact the medicament suspension may be coated with materials such as fluoropolymer materials which reduce the tendency of medicament to adhere thereto. Any movable parts may also. have coatings applied thereto which enhance their desired movement characteristics. Frictional coatings may therefore be applied to enhance frictional contact and lubricants used to reduce frictional contact as necessary.

It will be understood that the present disclosure is for the purpose of illustration only and the invention extends to modifications, variations and improvements thereto within the scope of the appended claims.

## Claims

1. An inhaler for delivery of a dry powder medicament, the inhaler comprising: a reservoir (720) for said dry powder; and a meter (722) for metering an amount of dry powder from said reservoir; **characterized by** a breath sensor (780) for sensing the breath of a patient; an electro-mechanical coupling means (726, 728, 730) for actuating said meter, wherein said coupling means is directly or indirectly responsive to said breath sensor; and dose-release means (786) which comprises an active dose-release mechanism which comprises means to propel pressurised gas in the direction of patient inhalation.

2. An inhaler according to claim 1 wherein the meter comprises a volume and/or a weight and/or a time and/or a surface-area and/or a particle counting regulated mechanism.

3. An Inhaler according to claim 1 or claim 2 wherein the meter comprises a valve (for example, a linear or rotary valve) and/or a piston and/or a load cell and/or a plunger.

4. An inhaler according to any one of the preceding claims wherein the meter comprises at least one metering chamber.

5. An inhaler according to claim 4 wherein on actuation of the meter, the or each metering chamber moves into fluid communication with the reservoir.

6. An inhaler according to claim 4 wherein the meter and the reservoir are relatively rotatable with respect to each other about a common central axis.

7. An inhaler according to claim 6 wherein the or each metering chamber is adapted to be In fluid communication selectively with the reservoir or with the patient

8. An inhaler according to any one of claims 4 to 7 wherein the or each metering chamber has a variable volume.

9. An inhaler according to any one of claims 4 to 7 wherein the or each metering chamber has a fixed volume which metering volume is variable by insertion of a plunger or piston.

10. An inhaler according to any one of claims 4 to 9 wherein the or each metering chamber is formed from expandable material.

11. An inhaler according to any one of claims 4 to 9 wherein the or each metering chamber has a telescopic or concertina arrangement.

12. An inhaler according to any one of claims 4 to 11 further comprising a gas permeable dry powder retaining means below the or each metering chamber.

13. An inhaler according to claim 12 wherein the retaining means is made from a gas-permeable filter, a mesh screen, a porous material or a perforated chamber element

14. An inhaler according to any one of the preceding claims, additionally comprising a reset mechanism for resetting the meter after actuation thereof.

15. An Inhaler according to any one of the preceding claims further comprising transport means to transport the metered volume from the reservoir to a delivery position.

16. An inhaler according to claim 15 wherein the transport means is actuable by the meter.

17. An inhaler according to any one of claims 1 to 16 wherein the dose-release means is actuable by the coupling means and/or the meter and/or the transport means.

18. An inhaler according to any one of the preceding claims wherein the gas-propelling means provides at least one pulse of gas on actuation.

19. An inhaler according to any one of the preceding claims wherein the gas-propelling means provides one pulse of gas for each dose dispensed.

20. An inhaler according to any one of claims 1 to 19 wherein the gas is air.

21. An inhaler according to any one of claims 1 to 19 wherein the gas is an inert gas.

22. An inhaler according to any one of the preceding claims additionally comprising climate control means.

23. An inhaler according to claim 22 wherein the climate control means is actuable by the coupling means and/or the meter and/or the transport means and/or the release means.

24. An inhaler according to claim 23 wherein the climate control means comprises means to (i) reduce moisture increase in the inhaler; and/or (ii) maintain ambient temperature; and/or (iii) dry the meter prior to actuation of the inhaler.

25. An inhaler according to any one of claims 22 to 24 wherein the climate control means comprises a desiccant.

26. An inhaler according to any one of claims 22 to 25 wherein the climate control means comprises a heater.

27. An inhaler according to claim 26 wherein the heater dries the meter , prior to metering of the volume and/or immediately after the volume is released.

28. An inhaler according to any one of claims 22 to 27 wherein the climate control means comprises a temperature and/or a moisture sensor.

29. An inhaler according to any one of the preceding claims wherein the coupling means comprises a spring and/or a lever.

30. An inhaler according to any one of the preceding claims wherein the coupling means comprises a solenoid.

31. An inhaler as claimed in any one of the preceding claims wherein the coupling means is reversibly deformable in response to heating thereof or application of a magnetic field thereto.

32. An inhaler according to claim 31, additionally comprising a reset coupling which is reversibly deformable in response to heating thereof or application of a magnetic field thereto.

33. An inhaler according to claim 31 or 32, wherein the heating is achievable by electric current flow through the coupling.

## Patentansprüche

1. Inhalator zur Abgabe eines Trockenpulvermedikaments, wobei der Inhalator umfasst:
ein Reservoir (720) für das Trockenpulver; und eine Messvorrichtung (722) zum Abmessen einer Menge von Trockenpulver aus dem Reservoir; **gekennzeichnet durch**
einen Atemsensor (780) zum Wahrnehmen des Atems von einem Patienten, eine elektro-mechanische Kopplungseinrichtung (726, 728, 730) zum Betätigen der Messvorrichtung, wobei die Kopplungseinrichtung direkt oder indirekt auf den Atemsensor anspricht; und eine Dosis-Freigabeeinrichtung (786), die einen aktiven Dosis-Freigabemechanismus umfasst, welcher eine Einrichtung umfasst, um unter Druck stehendes Gas in die Richtung der Patienteninhalation zu treiben.

2. Inhalator nach Anspruch 1, bei dem die Messvorrichtung einen Volumen- und/oder einen Gewichts- und/oder einen Zeit- und/oder einen Oberflächenbereich- und/oder einen Partikelzähl-geregelten Mechanismus umfasst.

3. Inhalator nach Anspruch 1 oder 2, bei dem die Messvorrichtung ein Ventil (zum Beispiel ein Linearventil oder einen Drehschieber) und/oder einen Kolben und/oder eine Messdose und/oder einen Stempel umfasst.

4. Inhalator nach_einem der vorhergehenden Ansprüche, bei dem die Messvorrichtung zumindest eine Messkammer umfasst.

5. Inhalator nach Anspruch 4, bei dem sich, auf eine Betätigung der Messvorrichtung hin, die oder jede Messkammer in Fluidverbindung mit dem Reservoir bewegt.

6. Inhalator nach Anspruch 4, bei dem die Messvorrichtung und das Reservoir, um eine gemeinsame Mittelachse, relativ drehbar bezüglich einander sind.

7. Inhalator nach Anspruch 6, bei dem die oder jede Messkammer geeignet ist, selektiv mit dem Reservoir oder mit dem Patienten in Fluidverbindung zu sein.

8. Inhalator nach einem der Ansprüche 4 bis 7, bei dem die oder jede Messkammer ein variables Volumen aufweist.

9. Inhalator nach einem der Ansprüche 4 bis 7, bei dem die oder jede Messkammer ein festes Volumen aufweist, wobei das Messvolumen durch ein Einführen von einem Stempel oder Kolben variabel ist.

10. Inhalator nach einem der Ansprüche 4 bis 9, bei dem die oder jede Messkammer aus einem aufweitbaren Material ausgebildet ist.

11. Inhalator nach einem der Ansprüche 4 bis 9, bei dem die oder jede Messkammer eine Teleskop- oder Konzertina-Anordnung aufweist.

12. Inhalator nach einem der Ansprüche 4 bis 11, ferner mit einer gasdurchlässigen Trockenpulver-Halteeinrichtung unterhalb der oder jeder Messkammer.

13. Inhalator nach Anspruch 12, bei dem die Halteeinrichtung aus einem gasdurchlässigen Filter, einem Maschensieb, einem porösen Material oder einem perforierten Kammerelement hergestellt ist.

14. Inhalator nach einem der vorhergehenden Ansprüche, zusätzlich mit einem Rückstellmechanismus zum Zurückstellen der Messvorrichtung nach einer Betätigung von ihr.

15. Inhalator nach einem der vorhergehenden Ansprüche, ferner mit einer Transporteinrichtung, um das abgemessene Volumen von dem Reservoir in eine Abgabeposition zu transportieren.

16. Inhalator nach Anspruch 15, bei dem die Transporteinrichtung durch die Messvorrichtung betätigbar ist.

17. Inhalator nach einem der Ansprüche 1 bis 16, bei dem die Dosis-Freigabeeinrichtung durch die Kopplungseinrichtung und/oder die Messvorrichtung und/oder die Transporteinrichtung betätigbar ist.

18. Inhalator nach einem der vorhergehenden Ansprüche, bei dem die Gas-Treibeinrichtung zumindest einen Gasimpuls auf eine Betätigung hin vorsieht.

19. Inhalator nach einem der vorhergehenden Ansprüche, bei dem die Gas-Treibeinrichtung einen Gasimpuls für jede abgegebene Dosis vorsieht.

20. Inhalator nach einem der Ansprüche 1 bis 19, bei dem das Gas Luft ist.

21. Inhalator nach einem der Ansprüche 1 bis 19, bei dem das Gas ein Inertgas ist.

22. Inhalator nach einem der vorhergehenden Ansprüche, zusätzlich mit einer Klimasteuereinrichtung.

23. Inhalator nach Anspruch 22, bei dem die Klimasteuereinrichtung durch die Kopplungseinrichtung und/oder die Messvorrichtung und/oder die Transporteinrichtung und/oder die Freigabeeinrichtung betätigbar ist.

24. Inhalator nach Anspruch 23, bei dem die Klimasteuereinrichtung Einrichtungen umfasst, um (i) eine Feuchtigkeitszunahme in dem Inhalator zu verringern; und/oder (ii) eine Umgebungstemperatur beizubehalten; und/oder (iii) die Messvorrichtung vor einer Betätigung des Inhalators zu trocknen.

25. Inhalator nach einem der Ansprüche 22 bis 24, bei dem die Klimasteuereinrichtung ein Trockenmittel umfasst.

26. Inhalator nach einem der Ansprüche 22 bis 25, bei dem die Klimasteuereinrichtung eine Heizvorrichtung umfasst.

27. Inhalator nach Anspruch 26, bei dem die Heizvorrichtung die Messvorrichtung vor einem Abmessen des Volumens, und/oder unmittelbar nach dem das Volumen freigegeben wird, trocknet.

28. Inhalator nach einem der Ansprüche 22 bis 27, bei dem die Klimasteuereinrichtung einen Temperatur- und/oder einen Feuchtigkeitssensor umfasst.

29. Inhalator nach einem der vorhergehenden Ansprüche, bei dem die Kopplungseinrichtung eine Feder und/oder einen Hebel umfasst.

30. Inhalator nach einem der vorhergehenden Ansprüche, bei dem die Kopplungseinrichtung ein Solenoid umfasst.

31. Inhalator nach einem der vorhergehenden Ansprüche, bei dem die Kopplungseinrichtung als Antwort auf ein Erwärmen davon, oder einem Anlegen eines Magnetfelds daran, reversibel verformbar ist.

32. Inhalator nach Anspruch 31, zusätzlich mit einer Rückstellkopplung, die als Antwort auf ein Erwärmen davon, oder einem Anlegen eines Magnetfelds daran, reversibel verformbar ist.

33. Inhalator nach Anspruch 31 oder 32, bei dem das Erwärmen durch einen elektrischen Stromfluss durch die Kopplung erreichbar ist.

## Revendications

1. Inhalateur destiné à administrer un médicament en poudre sèche, l'inhalateur comprenant : un réservoir (720) pour ladite poudre sèche ; et un dispositif de mesure (722) destiné à mesurer une quantité de poudre sèche à partir dudit réservoir ; **caractérisé par** un capteur de respiration (780) destiné à détecter la respiration d'un patient ; des moyens de couplage électromécaniques (726, 728, 730) destinés à actionner ledit dispositif de mesure, dans lequel lesdits moyens de couplage réagissent directement ou indirectement au dit capteur de respiration ; et des moyens de libération de dose (786) qui comprennent un mécanisme de libération de dose actif, qui comprennent des moyens pour propulser un gaz sous pression dans le sens d'inhalation du patient.

2. Inhalateur selon la revendication 1, dans lequel le dispositif de mesure comprend un mécanisme régulé de comptage de volume et/ou de poids et/ou de temps et/ou de zone de surface et/ou de particules.

3. Inhalateur selon la revendication 1 ou la revendication 2, dans lequel le dispositif de mesure comprend une soupape (par exemple une soupape linéaire ou rotative) et/ou un piston et/ou une cellule de chargement et/ou un piston plongeur.

4. Inhalateur selon l'une quelconque des revendications précédentes, dans lequel le dispositif de mesure comprend au moins une chambre de mesure.

5. Inhalateur selon la revendication 4, dans lequel lors de l'actionnement du dispositif de mesure, la ou chaque chambre de mesure se déplace en communication de fluide avec le réservoir.

6. Inhalateur selon la revendication 4, dans lequel le dispositif de mesure et le réservoir sont relativement rotatifs l'un par rapport à l'autre autour d'un axe central commun.

7. Inhalateur selon la revendication 6, dans lequel la ou chaque chambre de mesure est adaptée pour être en communication de fluide de façon sélective avec le réservoir ou avec le patient.

8. Inhalateur selon l'une quelconque des revendications 4 à 7, dans lequel la ou chaque chambre de mesure présente un volume variable.

9. Inhalateur selon l'une quelconque des revendications 4 à 7, dans lequel la ou chaque chambre de mesure présente un volume fixé lequel volume de mesure est variable par insertion d'un plongeur ou d'un piston.

10. Inhalateur selon l'une quelconque des revendications 4 à 9, dans lequel la ou chaque chambre de mesure est formée à partir d'un matériau extensible.

11. Inhalateur selon l'une quelconque des revendications 4 à 9, dans lequel la ou chaque chambre de mesure présente un agencement télescopique ou en accordéon.

12. Inhalateur selon l'une quelconque des revendications 4 à 11, comprenant en outre des moyens de retenue de poudre sèche perméables au gaz au-dessous de la ou chaque chambre de mesure.

13. Inhalateur selon la revendication 12, dans lequel les moyens de retenue sont composés d'un filtre perméable au gaz, un tamis à mailles, un matériau poreux ou un élément de chambre perforé.

14. Inhalateur selon l'une quelconque des revendications précédentes, comprenant en outre un mécanisme de réinitialisation destiné à réinitialiser le dispositif de mesure après son actionnement.

15. Inhalateur selon l'une quelconque des revendications précédentes, comprenant en outre des moyens de transport destinés à transporter le volume mesuré à partir du réservoir vers une position d'administration.

16. Inhalateur selon la revendication 15, dans lequel les moyens de transport sont actionnables par le dispositif de mesure.

17. Inhalateur selon l'une quelconque des revendications 1 à 16 dans lequel les moyens de libération de dose peuvent être actionnés par les moyens de couplage et/ou le dispositif de mesure et/ou les moyens de transport.

18. Inhalateur selon l'une quelconque des revendications précédentes dans lequel les moyens de propulsion de gaz fournissent au moins une impulsion de gaz lors de l'actionnement.

19. Inhalateur selon l'une quelconque des revendications précédentes dans lequel les moyens de propulsion de gaz fournissent une impulsion de gaz pour chaque dose distribuée.

20. Inhalateur selon l'une quelconque des revendications 1 à 19, dans lequel le gaz est l'air.

21. Inhalateur selon l'une quelconque des revendications 1 à 19, dans lequel le gaz est un gaz inerte.

22. Inhalateur selon l'une quelconque des revendications précédentes comprenant en outre des moyens de conditionnement d'air.

23. Inhalateur selon la revendication 22, dans lequel les moyens de conditionnement d'air peuvent être actionnés par les moyens de couplage et/ou le dispositif de mesure et/ou les moyens de transport et/ou les moyens de libération.

24. Inhalateur selon la revendication 23 dans lequel les moyens de conditionnement d'air comprennent des moyens pour (i) réduire l'augmentation d'humidité dans l'inhalateur ; et/ou (ii) maintenir la température ambiante ; et/ou (iii) sécher le dispositif de mesure avant l'actionnement de l'inhalateur.

25. Inhalateur selon l'une quelconque des revendications 22 à 24 dans lequel les moyens de conditionnement d'air comprennent un déshydratant.

26. Inhalateur selon l'une quelconque des revendications 22 à 25, dans lequel les moyens de conditionnement d'air comprennent un dispositif de chauffage.

27. Inhalateur selon la revendication 26, dans lequel le dispositif de chauffage sèche le dispositif de mesure avant de mesurer le volume et/ou immédiatement après que le volume est libéré.

28. Inhalateur selon l'une quelconque des revendications 22 à 27, dans lequel les moyens de conditionnement d'air comprennent un capteur dé température et/ou d'humidité.

29. Inhalateur selon l'une quelconque des revendications précédentes, dans lequel les moyens de couplage comprennent un ressort et/ou un levier.

30. Inhalateur selon l'une quelconque des revendications précédentes, dans lequel les moyens de couplage comprennent un solénoïde.

31. Inhalateur selon l'une quelconque des revendications précédentes, dans lequel les moyens de couplage sont déformables de façon réversible en réponse à leur chauffage ou à l'application d'un champ magnétique sur ceux-ci.

32. Inhalateur selon la revendication 31, comprenant en outre un couplage de réinitialisation qui est déformable de façon réversible en réponse au chauffage de celui-ci ou à l'application d'un champ magnétique sur celui-ci.

33. Inhalateur selon la revendication 31 ou 32, dans lequel le chauffage peut être atteint par un flux de courant électrique à travers le couplage.
